(19) Europäisches Patentamt
European Patent Office
Office européen des brevets

(11) **EP 4 206 219 A1**

(12) **EUROPEAN PATENT APPLICATION**
published in accordance with Art. 153(4) EPC

(43) Date of publication:
**05.07.2023 Bulletin 2023/27**

(21) Application number: **21862145.6**

(22) Date of filing: **30.08.2021**

(51) International Patent Classification (IPC):
*C07K 14/54* [(2006.01)]  *C07K 14/715* [(2006.01)]
*C07K 14/55* [(2006.01)]  *C12N 5/0783* [(2010.01)]
*A61K 35/17* [(2015.01)]  *A61P 35/00* [(2006.01)]
*A61P 31/00* [(2006.01)]

(52) Cooperative Patent Classification (CPC):
**A61K 35/17; A61P 31/00; A61P 35/00;
C07K 14/54; C07K 14/55; C07K 14/715; C12N 5/06**

(86) International application number:
**PCT/KR2021/011575**

(87) International publication number:
**WO 2022/045849 (03.03.2022 Gazette 2022/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2020 KR 20200109995
27.08.2021 KR 20210114086**

(71) Applicant: **GI Cell, Inc.
Seongnam-si, Gyeonggi-do 13201 (KR)**

(72) Inventors:
• **JANG, Myoung Ho
Seoul 05849 (KR)**

• **HONG, Chun-Pyo
Seongnam-si Gyeonggi-do 13643 (KR)**
• **YANG, Zung Yoon
Incheon 22009 (KR)**
• **KO, Dongwoo
Seoul 06601 (KR)**
• **LEE, June Sub
Gwangju-si Gyeonggi-do 12795 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **FUSION PROTEIN INCLUDING IL15 PROTEIN, IL15 RECEPTOR ALPHA PROTEIN, FC DOMAIN, AND IL2 PROTEIN, AND USE THEREOF**

(57) The present invention relates to a fusion protein including an IL15 protein, an IL15 receptor alpha (IL15Rα) protein, an Fc domain, and an IL2 protein, and a use thereof, and more specifically, to: a fusion protein including an IL15 protein, an IL15Rα protein, an Fc domain, and an IL2 protein; a dimer including the fusion protein; a composition that is for culturing natural killer (NK) cells and includes the fusion protein or dimer; and a method for culturing NK cells. The fusion protein according to the present invention makes it possible to efficiently proliferate and culture NK cells while maintaining the cytolytic activity of the NK cells and promoting the activation of the NK cells when culturing the NK cells, thus making it possible to obtain a large number of NK cells without using feeder cells. Therefore, the fusion protein is useful for the commercialization of NK cells into safe cell therapy agents.

FIG. 1

**Description**

**TECHNICAL FIELD**

The present invention relates to a fusion protein comprising an IL15 protein, an IL15 receptor alpha (IL15Rα) protein, an Fc domain and an IL2 protein, and the uses thereof, and more particularly to a fusion protein comprising an IL15 protein, an IL15Rα protein, an Fc domain and an IL2 protein, a dimer comprising the fusion protein, a composition for culturing natural killer cells (NK cells) comprising the fusion protein or the dimer and a method for culturing NK cells.

**BACKGROUND ART**

NK cells (natural killer cells) are lymphocyte-based cells that play a crucial role in the immune response. NK cells are considered to be an essential member of an immune surveillance mechanism for removing abnormal cells that are tumorigenic or undergo tumorigenesis *in vivo.* Therefore, research has been conducted for a long time on the effective use of these cells for the treatment of tumors and for the removal of virus-infected cells, which are presumed to be the source of tumors.

A sufficiently large dose of cells and repeated administration are very important factors for cell therapy. However, failure to administrate a sufficient number of cells resulting from insufficient proliferation of NK cells has been the biggest obstacle to clinical application. The number of NK cells present in the body is not large, so technology for mass-producing NK cells while maintaining sufficient efficacy of NK cells to an extent to which the NK cells are useful for therapeutic purposes is required. However, NK cells do not properly proliferate *in vitro,* and are thus difficult to culture in large amounts. Therefore, attention and a great deal of research is focused on amplifying and culturing NK cells to a level that is useful in practice, but the technology is still inapplicable to clinical practice.

The development of technology for *in vitro* culture and amplification of NK cells obtained from a donor to obtain NK cells capable of killing target cells is in progress. There have been studies on the proliferation of NK cells using IL2, previously used for T cell proliferation/activation, IL15 (Dunne J et al., Immunology, vol. 167:3129. 2001), LPS (MR Goodier et al., Immunology 165:139, 2000), an OKT-3 antibody stimulating CD3 (Condiotti R., et al., Experimental Hematology 29:104, 2001), and combinations thereof. However, these studies only identify novel materials obtained through modification and development of traditionally used IL2, and do not suggest a breakthrough proliferation method.

An effective method of improving NK cell viability and *in vitro* proliferation is a method of co-culturing NK cells with feeder cells. Feeder cells commonly used for NK proliferation include irradiated PBMCs, Epstein-Barr virus transformed lymphoblastic cell line (EBC-LCL), genetically modified K562 cells that express the structure of IL15 or 21, and other irradiated tumor cell lines (Berg, M., et al., Cytotherapy. 2009; 11(3):341-55; Baek, H. J., et al., Anticancer Res. 2013; 33(5):2011-9). Co-culture with feeder cells improves NK cell viability and increases proliferation 1,000 to 50,000-fold (Denman, C. J., et al., PLoS ONE. 2012;7(1): e30264).

Meanwhile, cases in which NK cells are amplified using a tumor cell line as feeder cells have been reported. Although CTV-1, a leukemia cell line, was used as feeder cells, there was little improvement in proliferation (J. Immunol., 178(1): p85-94, 2007), and a 490-fold increase in the number of NK cells when cultured for 21 days using EBV-LCL has been reported (Cytotherapy, 11(3): p341-355, 2009). However, all of these results were obtained using methods that are unsuitable for ensuring safety, which is important for clinical applications, such as the use of cancer cell lines as feeder cells, and these methods have a limitation in that NK cells imparted with priming specificity to certain cancer cells are produced using a specific cancer cell line rather than normal cells as feeder cells.

Accordingly, various attempts have been made to culture NK cells using a "feeder cell free" method that does not use feeder cells. When cytokines such as IL2, IL12, IL15, IL18, IL21 or nicotinamide are provided in sufficient amounts, NK cells can be cultured without feeder cells. The produced NK cells exhibit increased cytotoxicity compared to freshly isolated NK cells, but can only proliferate up to 100 to 300 times the original number thereof (Childs, R. W. et al., Hematology Am. Soc. Hematol. Educ. Program. 2013;2013(1):234-46). This limited proliferation is due to telomere loss and aging (Denman, C. J., et al., PLoS ONE. 2012;7(1):e30264). Residual IL2 may also cause serious side effects in patients (Ni, J., et al., OncoImmunology 2013; 2:4, e23811) .

In addition, NK cells were cultured using IL15 and IL15 receptor alpha (IL15Rα) (US 8,124,084 B), or using IL2, IL15 (or IL12, IL18) and anti-NKp46 antibodies (JP 6073417 B) in order to increase the proliferation of immune cells including NK cells. However, the disclosed above also fails to obtain a remarkably increased NK cell proliferation.

Accordingly, as a result of extensive efforts to develop an NK cell culture method that is capable of further increasing the proliferation and survival rate of NK cells using a "feeder cell free" method, the present inventors found that by culturing NK cells using a fusion protein comprising an IL15 protein, an IL15 receptor alpha (IL15Rα) protein, an Fc domain and an IL2 protein, it is possible to remarkably increase the proliferation and cell survival of NK cells and effectively promote the activation of NK cells while maintaining the killing ability of NK cells, and completed the present invention based thereon.

The information disclosed in this Background section is provided only for better understanding of the background of the present invention, and therefore it may not include information that forms the prior art that is already obvious to those skilled in the art.

**SUMMARY OF THE INVENTION**

It is an object of the present invention to provide a fusion protein that is capable of effectively culturing NK cells while ensuring safety.

It is another object of the present invention to provide a composition for culturing NK cells comprising the fusion protein and a method for culturing NK cells using the composition.

It is another object of the present invention to provide a composition for treating cancer, an immune disease, or an infectious disease comprising the NK cells prepared by the culturing method.

It is another object of the present invention to provide a method for preventing or treating cancer, an immune disease, or an infectious disease using the NK cells prepared by the culturing method, the use of the NK cells for the prevention or treatment of cancer, an immune disease, or an infectious disease, and the use of the NK cells for the preparation of a therapeutic agent for preventing or treating cancer, an immune disease, or an infectious disease.

In order to achieve the above objects, the present invention provides a fusion protein comprising an IL15 protein, an IL15 receptor alpha (IL15Rα) protein, an Fc domain, and an IL2 protein.

In addition, the present invention provides a polynucleotide encoding the fusion protein, a vector comprising the polynucleotide, a transformed cell introduced with the vector, and a method for producing a fusion protein comprising culturing the transformed cell.

In addition, the present invention provides a fusion protein dimer in which two fusion proteins comprising the fusion protein are bound to each other.

In addition, the present invention provides a composition for culturing natural killer cells (NK cells) comprising the fusion protein or fusion protein dimer, and a method of culturing NK cells (natural killer cells) comprising culturing NK cells in a medium containing the composition.

In addition, the present invention provides a composition for treating cancer, an immune disease, or an infectious disease comprising the NK cells prepared by the culturing method.

In addition, the present invention provides a method for preventing or treating cancer, an immune disease, or an infectious disease administering the NK cells prepared by the culturing method, the use of the NK cells for prevention or treatment of cancer, an immune disease, or an infectious disease, and the use of the NK cells for preparation of a therapeutic agent for preventing or treating cancer, an immune disease, or an infectious disease.

**BRIEF DESCRIPTION OF THE DRAWINGS**

FIG. 1 is a schematic diagram illustrating the structure of a fusion protein according to the present invention.

FIG. 2 illustrates the results of D0 FACS analysis of CD3- CD56+ NK cells isolated from PBMCs.

FIG. 3 is a graph illustrating a comparison of the survival rate and proliferation rate of CD3- CD56+ PBMC cells cultured for 18 days using Comparative Examples (1, 2, and 3) and fusion proteins according to the present invention (Experimental Examples 1-1, 1-2, 1-3, 2-1, 2-2, and 2-3).

FIGS. 4 to 6 are graphs illustrating purity (FIG. 4), an active marker, an inhibitory marker, and a marker involved in cancer killing (FIGS. 5 and 6) identified through flow cytometry with respect to the cells cultured for 18 days using Comparative Examples (1, 2, and 3) and fusion proteins according to the present invention (Experimental Examples 1-1, 1-2, 1-3, 2-1, 2-2, and 2-3).

**DETAILED DESCRIPTION OF THE INVENTION**

Unless defined otherwise, all technical and scientific terms used herein have the same meanings as appreciated by those skilled in the field to which the present invention pertains. In general, the nomenclature used herein is well-known in the art and is ordinarily used.

Immunotherapy, which has recently emerged, is a treatment method that minimizes damage to normal cells and removes cancer cells with greater specificity using the human's own immune system. Research in several particular fields (antibody therapy, immune cell therapy, viral immunotherapy, nanotechnology immunotherapy, and the like) is being actively conducted. Among them, immune cell therapy is a method for treating cancer by increasing the number of natural killer cells (NK cells), natural killer T cells, T cells, B cells, dendritic cells, and the like, among lymphocytes obtained from the patient's blood, enhancing the functions *in vitro,* and reintroducing the cells into the patient. This treatment method using immune cells is expected to exhibit excellent immune response modulation effects and superior toxicity and safety. Among them, NK cells are essential cells responsible for innate immunity, and mature in the liver and bone marrow, unlike T cells. In particular, NK cells have a function of self-identifying and killing abnormal cells such as virus-infected cells or tumor cells.

There is need for a method for massively proliferating NK cells that are capable of overcoming immune rejection and providing patient-specific cell therapy as a cell therapy agent for the treatment of intractable diseases such as cancer, and a method for culturing NK cells with improved activity.

In one embodiment of the present invention, when culturing NK cells using a fusion protein comprising an IL15 protein, an IL15 receptor alpha (IL15Rα) protein, an Fc domain, and an IL2 protein, and a composition for culture comprising the same, it is possible to remarkably increase the proliferation and cell survival of NK cells and effectively promote the activation of NK cells, while maintaining the killing ability of NK cells, compared to when culturing NK cells using a conventional known NK cell culture method using cytokines such as IL2. This suggests that NK cells can be cultured more effectively and that safety of the NK cells can be secured because NK cells are cultured using a "feeder cell free" method, so the NK cells can be useful as a cell therapy agent.

Accordingly, in one aspect, the present invention is directed to a fusion protein comprising an IL15 protein, an IL15 receptor alpha (IL15Rα) protein, an Fc domain, and an IL2 protein.

In the present invention, the IL15 protein may comprise the amino acid sequence represented by SEQ ID NO: 1, and the IL15 receptor alpha (IL15Rα) protein may comprise the amino acid sequence represented by SEQ ID NO: 3, but the present invention is not limited thereto.

Interleukin 15 (IL15) is a cytokine that is structurally similar to IL2 secreted by mononuclear phagocytes (and several other cells) after infection with a virus or after indirect stimulation by cells that are inactivated or recognized as non-self. IL15 and IL2 exert cellular signaling functions through binding to a trimeric complex composed of two shared receptors, namely the common gamma chain (γc, CD132) and the IL2 receptor B chain (IL-2Rβ, CD122), as well as the IL2 receptor alpha (IL2Rα; CD25) or IL15 receptor alpha (IL15Rα; CD215), which is the alpha chain receptor that is unique to each cytokine.

As is well-known in the art, the IL15Ra protein contains the "sushi domain", the shortest region of the receptor that retains IL15 binding activity. Accordingly, a fusion protein comprising the entire IL15Rα protein may be prepared, but a fusion protein comprising a sushi domain is preferably prepared.

As used herein, the term "IL15 protein" refers to a full-length IL15 or IL15 fragment, and the term "IL15Rα protein" refers to a full-length IL15Rα or IL15Rα fragment.

As used herein, the term "IL15 fragment" or "IL15Rα fragment" refers to cleaved IL15 or IL15Rα, and the IL15Rα fragment may be a sushi domain of IL15Rα.

In the present invention, the Fc domain may comprise a heavy-chain constant region 2 (CH2) and a heavy-chain constant region 3 (CH3) of immunoglobulin.

In the present invention, the Fc (heavy-chain constant region) domain may further comprise a hinge domain.

In the present invention, the immunoglobulin may be IgG, IgA, IgE, IgD, or IgM.

Preferably, the Fc domain may be an IgG Fc domain, for example, an IgG1, IgG2, IgG3 or IgG4 Fc domain, and more preferably an IgG4 Fc domain, but is not limited thereto.

In addition, the Fc domain of the immunoglobulin may be a wild type Fc domain as well as an Fc domain variant. In addition, as used herein, the term "Fc domain variant" may have a glycosylation pattern different from that of the wild type Fc domain, may have a greater number of glycans compared to the wild type Fc domain, may have a smaller number of glycans compared to the wild type Fc domain, or may have a deglycosylated form from which the glycans are removed from the wild type Fc domain. The term "Fc domain variant" may include an aglycosylated Fc domain. The Fc domain or variant may have sialylation, fucosylation, and glycosylation levels which are adjusted through culture conditions or genetic manipulation of the host.

In the present invention, the Fc domain may comprise the amino acid sequence represented by SEQ ID NO: 5, but is not limited thereto.

In the present invention, the IL2 protein may be a wild type or a variant.

Interleukin 2 (IL2), which is also called a T-cell growth factor (TCGF), is a globular glycoprotein that plays a key role in lymphocyte production, survival, and homeostasis. IL2 has a protein size of 15.5 kDa to 16 kDa and consists of 133 amino acids. IL2 mediates various immune actions by binding to the IL2 receptor, which is composed of three individual subunits.

In addition, IL2 is mainly synthesized by CD4+ helper T cells, among activated T cells. IL2 stimulates proliferation and differentiation of T cells, induces production of cytotoxic T lymphocytes (CTLs), and differentiation of peripheral blood lymphocytes into cytotoxic cells and lymphokine activated killer cells (LAK cells).

As used herein, the term "IL2 protein" means a full-length IL2 or IL2 fragment, and the term "IL2 fragment" means cleaved IL2.

In the present invention, the wild type IL2 protein may comprise the amino acid sequence represented by SEQ ID NO: 7.

In the present invention, the IL2 protein variant may comprise the amino acid sequence represented by SEQ ID NO: 8 having R38A and F42A mutations in the amino acid sequence of SEQ ID NO: 7, but is not limited thereto.

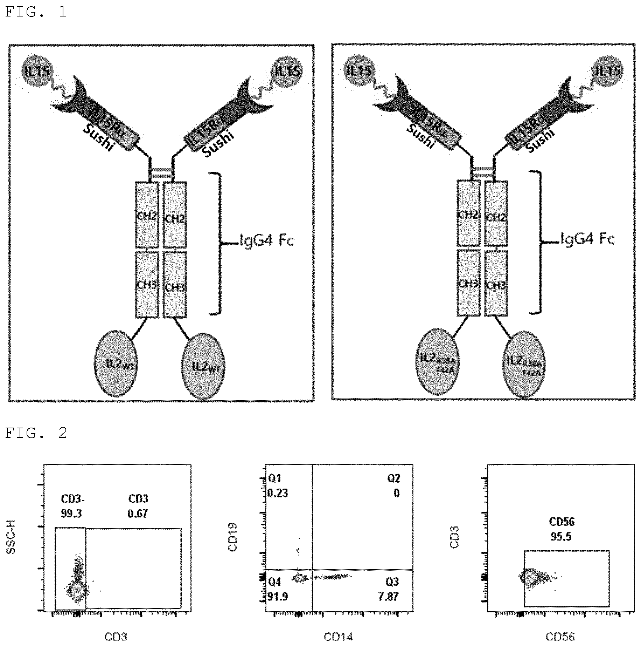

In the present invention, the fusion protein may have a structure in which IL15Rα linked with IL5 directly or via a linker, and IL2 are bound to the N-terminus and C-terminus, respectively, of an Fc domain, or IL2 and IL15Rα linked with IL5 directly or via a linker are bound to the N-terminus and C-terminus, respectively, of an Fc domain (FIG. 1). The N-terminus or C-terminus of the Fc domain to IL15Rα or IL2 may optionally be connected via a linker peptide.

Specifically, the fusion protein may comprise the following Formula (I):

N'-X-[Linker(1)]1-Y-[Linker(2)]n-Fc domain-[Linker(3)]m-Z-C' (Formula (I))

Wherein,

N' is the N-terminus of the fusion protein, and C' is the C-terminus of the fusion protein,
X is an IL15 protein, Y is an IL15 receptor alpha (IL15Rα) protein, Z is an IL2 protein,
Linker (1), Linker (2) and Linker (3) are peptide linkers, and
I, n, and m are each independently 0 or 1.

In the present invention, the linker connects two proteins to each other. In one embodiment, the linker may include 1 to 100 amino acids, albumin or a fragment thereof, an Fc hinge domain of an immunoglobulin or the like.

In the present invention, the linker (1) and linker (3) may comprise 1 to 100 consecutive amino acids, 5 to 50 consecutive amino acids, or 5 to 30 amino acids. In one embodiment of the present invention, the linker (1) and the linker (3) may be (G4S)n, wherein n is an integer of 1 to 10, that is, 1, 2, 3, 4, 5, 6, 7, 8, 9, or 10, but is not limited thereto.

In the present invention, the linker (2) may comprise 1 to 100 consecutive amino acids, 5 to 80 consecutive amino acids, 20 to 60 consecutive amino acids, or 25 to 50 consecutive amino acids. In one embodiment of the present invention, the linker (2) may comprise (G4S)n and an immunoglobulin hinge, wherein n is an integer from 1 to 10, that is, 1, 2, 3, 4, 5, 6, 7, 8, 9 or 10, but is not limited thereto.

In the present invention, preferably, the linker (1) comprises the amino acid sequence represented by SEQ ID NO: 2, the linker (2) comprises the amino acid sequence represented by SEQ ID NO: 4, and the linker (3) comprises the amino acid sequence represented by SEQ ID NO: 6, but the present invention is not limited thereto.

In the present invention, the fusion protein may comprise the amino acid sequence represented by SEQ ID NO: 9 or SEQ ID NO: 10.

In the present specification, the fusion protein comprising IL15, IL15Rα, an Fc domain, and an IL2 wild type (WT) according to the present invention is referred to as "fusion protein A" (Table 1), and the fusion protein comprising IL15, IL15Rα, an Fc domain and an IL2 variant is referred to as "fusion protein B" (Table 2).

[Table 1]

| SEQ. ID NO: | Component | Sequence | Number of amino acids |
|---|---|---|---|
| 13 | Albumin Signal Sequence | MKWVTFISLLFLFSSAYS | 18 |
| 1 | Human IL15 | NWVNVISDLKKIEDLIQSMHIDATLYTESDVHPSCKVTA MKCFLLELQVISLESGDASIHDTVENLIILANNSLSSNG NVTESGCKECEELEEKNIKEFLQSFVHIVQMFINTS | 114 (N-Gly site: 1) |
| 2 | Linker 1 | GGGGSGGGGSGGGGSGGGGS | 20 |
| 3 | Human IL15Ra Sushi domain | ITCPPPMSVEHADIWVKSYSLYSRERYICNSGFKRKAGT SSLTECVLNKATNVAHWTTPSLKCIRDPALVHQRPAPPS | 78 (N-Gly site: 1) |
| 4 | Linker 2 | GGGGSGGGGSGGGGSAESKYGPPCPPCP | 28 |

(continued)

| SEQ. ID NO: | Component | Sequence | Number of amino acids |
|---|---|---|---|
| 5 | IgG4 Fc | APEAAGGPSVFLFPPKPKDTLMISRTPEVTCVVVDVSQE DPEVQFNWYVDGVEVHNAKTKPREEQFNSTYRVVSVLTV LHQDWLNGKEYKCKVSNKGLPSSIEKTISKAKGQPREPQ VYTLPPSQEEMTKNQVSLTCLVKGFYPSDIAVEWESNGQ PENNYKTTPPVLDSDGSFFLYSRLTVDKSRWQEGNVFSC SVMHEALHNHYTQKSLSLSLG | 216 (N-Gly site: 1) |
| 6 | Linker 3 | GGGGS | 5 |
| 7 | Human IL2$_{WT}$ | APTSSSTKKTQLQLEHLLLDLQMILNGINNYKNPKLTRM LTFKFYMPKKATELKHLQCLEEELKPLEEVLNLAQSKNF HLRPRDLISNINVIVLELKGSETTFMCEYADETATIVEF LNRWITFCQSIISTLT | 133 (O-Gly site: 1) |
| 9 | Fusion Protein A | NWVNVISDLKKIEDLIQSMHIDATLYTESDVHPSCKVTA MKCFLLELQVISLESGDASIHDTVENLIILANNSLSSNG NVTESGCKECEELEEKNIKEFLQSFVHIVQMFINTSGGG GSGGGGSGGGGSGGGGSITCPPPMSVEHADIWVKSYSLY SRERYICNSGFKRKAGTSSLTECVLNKATNVAHWTTPSL KCIRDPALVHQRPAPPSGGGGSGGGGSGGGGSAESKYGP PCPPCPAPEAAGGPSVFLFPPKPKDTLMISRTPEVTCVV VDVSQEDPEVQFNWYVDGVEVHNAKTKPREEQFNSTYRV VSVLTVLHQDWLNGKEYKCKVSNKGLPSSIEKTISKAKG QPREPQVYTLPPSQEEMTKNQVSLTCLVKGFYPSDIAVE WESNGQPENNYKTTPPVLDSDGSFFLYSRLTVDKSRWQE GNVFSCSVMHEALHNHYTQKSLSLSLGGGGGSAPTSSST KKTQLQLEHLLLDLQMILNGINNYKNPKLTRMLTFKFYM PKKATELKHLQCLEEELKPLEEVLNLAQSKNFHLRPRDL ISNINVIVLELKGSETTFMCEYADETATIVEFLNRWITF CQSIISTLT | 594 (Monomer) |
| 11 | Fusion Protein A | GCCACCATGAAGTGGGTCACCTTCATCTCCCTGCTGTTT CTGTTCTCCTCCGCCTACTCCAACTGGGTCAACGTGATC TCCGACCTGAAGAAGATCGAGGACCTGATCCAGTCCATG CACATCGACGCTACCCTGTACACCGAGTCCGATGTGCAC CCTTCTTGCAAAGTGACCGCTATGAAGTGCTTCCTGCTG GAACTGCAAGTGATCTCCCTGGAATCCGGCGACGCCTCT ATCCACGATACCGTGGAAAACCTGATCATCCTGGCCAAC AACTCCCTGTCCTCCAACGGCAATGTGACCGAGTCTGGC TGCAAAGAGTGCGAGGAACTGGAAGAGAAGAACATCAAA GAGTTCCTCCAGTCCTTCGTCCACATCGTGCAGATGTTC ATCAACACCTCTGGCGGCGGAGGAAGTGGTGGCGGAGGA TCTGGCGGTGGTGGTTCTGGCGGAGGCGGATCTATTACC TGTCCACCTCCTATGTCCGTGGAACACGCCGACATCTGG GTCAAGTCCTACAGCCTGTACTCCAGAGAGCGGTACATC TGCAACTCCGGCTTCAAGAGAAAGGCCGGCACATCTTCT | |

(continued)

| SEQ. ID NO: | Component | Sequence | Number of amino acids |
|---|---|---|---|
| | | CTGACCGAGTGCGTGCTGAACAAGGCCACCAATGTGGCT<br>CACTGGACCACACCTAGCCTGAAGTGCATCAGAGATCCC<br>GCTCTGGTCCATCAGAGGCCTGCTCCTCCAAGCGGTGGT<br>GGTGGAAGCGGAGGTGGCGGATCAGGTGGTGGCGGTTCT<br>GCTGAGTCTAAGTACGGACCTCCTTGTCCACCTTGTCCT<br>GCTCCAGAAGCTGCTGGCGGACCAAGCGTTTTCCTGTTT<br>CCTCCAAAGCCTAAGGACACCCTGATGATCTCTCGGACC<br>CCTGAAGTGACCTGCGTGGTGGTCGATGTGTCTCAAGAG<br>GACCCCGAGGTGCAGTTCAATTGGTACGTGGACGGCGTG<br>GAAGTGCACAACGCCAAGACCAAGCCTAGAGAGGAACAG<br>TTCAACTCCACCTACAGAGTGGTGTCCGTGCTGACCGTG<br>CTGCACCAGGATTGGCTGAACGGCAAAGAGTACAAGTGC<br>AAGGTGTCCAACAAGGGCCTGCCTTCCAGCATCGAAAAG<br>ACCATCTCCAAGGCTAAGGGCCAGCCTCGGGAACCTCAG<br>GTTTACACCCTGCCTCCAAGCCAAGAGGAAATGACCAAG<br>AACCAGGTGTCCCTGACCTGCCTGGTCAAGGGCTTCTAC<br>CCTTCCGATATCGCCGTGGAATGGGAGTCCAATGGCCAG<br>CCTGAGAACAACTACAAGACAACCCCTCCTGTGCTGGAC<br>TCCGACGGCTCCTTTTTTCTGTACTCCCGCCTGACCGTG<br>GACAAGTCCAGATGGCAAGAGGGCAACGTGTTCTCCTGC<br>TCTGTGATGCACGAGGCCCTGCACAACCACTACACCCAG<br>AAGTCCCTGTCTCTGTCTCTTGGAGGCGGTGGCGGAAGT<br>GCTCCTACCTCCAGCTCTACCAAGAAAACCCAGCTGCAG<br>TTGGAGCATCTGCTGCTGGACCTCCAGATGATCCTGAAT<br>GGCATCAACAATTACAAGAACCCCAAGCTGACCCGGATG<br>CTGACCTTCAAGTTCTACATGCCCAAGAAGGCCACCGAG<br>CTGAAGCACCTCCAGTGCCTGGAAGAGGAACTGAAGCCC<br>CTGGAAGAAGTGCTGAATCTGGCCCAGTCCAAGAACTTC<br>CACCTGAGGCCTCGGGATCTGATCTCCAACATCAATGTG<br>ATCGTGTTGGAGCTGAAGGGCTCCGAGACAACCTTCATG<br>TGCGAGTACGCCGACGAGACAGCCACCATCGTGGAATTT<br>CTGAACCGGTGGATCACCTTCTGCCAGTCCATCATCTCC<br>ACACTGACCTGATGACTCGAGA | |

[Table 2]

| SEQ. ID NO: | Component | Sequence | Number of amino acids |
|---|---|---|---|
| 13 | Albumin Signal Sequence | MKWVTFISLLFLFSSAYS | 18 |
| 1 | Human IL15 | NWVNVISDLKKIEDLIQSMHIDATLYTESDVHPSCKVTA<br>MKCFLLELQVISLESGDASIHDTVENLIILANNSLSSNG<br>NVTESGCKECEELEEKNIKEFLQSFVHIVQMFINTS | 114 (N-Gly site: 1) |
| 2 | Linker 1 | GGGGSGGGGSGGGGSGGGGS | 20 |
| 3 | Human IL15Rα Sushi domain | ITCPPPMSVEHADIWVKSYSLYSRERYICNSGFKRKAGT<br>SSLTE<br>CVLNKATNVAHWTTPSLKCIRDPALVHQRPAPPS | 78 (N-Gly site: 1) |
| 4 | Linker 2 | GGGGSGGGGSGGGGSAESKYGPPCPPCP | 28 |

(continued)

| SEQ. ID NO: | Component | Sequence | Number of amino acids |
|---|---|---|---|
| 5 | IgG4 Fc | APEAAGGPSVFLFPPKPKDTLMISRTPEVTCVVVDVSQE DPEVQFNWYVDGVEVHNAKTKPREEQFNSTYRVVSVLTV LHQDWLNGKEYKCKVSNKGLPSSIEKTISKAKGQPREPQ VYTLPPSQEEMTKNQVSLTCLVKGFYPSDIAVEWESNGQ PENNYKTTPPVLDSDGSFFLYSRLT VDKSRWQEGNVFSCSVMHEALHNHYTQKSLSLSLG | 216 (N-Gly site: 1) |
| 6 | Linker 3 | GGGGS | 5 |
| 8 | Human IL2$_{R38A\ F42A}$ | APTSSSTKKTQLQLEHLLLDLQMILNGINNYKNPKLTAM LTAKFYMPKKATELKHLQCLEEELKPLEEVLNLAQSKNF HLRPRDLISNINVIVLELKGSETTFMCEYADETATIVEF LNRWITFCQSIISTLT | 133 (O-Gly site: 1) |
| 10 | Fusion Protein B | NWVNVISDLKKIEDLIQSMHIDATLYTESDVHPSCKVTA MKCFLLELQVISLESGDASIHDTVENLIILANNSLSSNG NVTESGCKECEELEEKNIKEFLQSFVHIVQMFINTSGGG GSGGGGSGGGGSGGGGSITCPPPMSVEHADIWVKSYSLY SRERYICNSGFKRKAGTSSLTECVLNKATNVAHWTTPSL KCIRDPALVHQRPAPPSGGGGSGGGGSGGGGSAESKYGP PCPPCPAPEAAGGPSVFLFPPKPKDTLMISRTPEVTCVV VDVSQEDPEVQFNWYVDGVEVHNAKTKPREEQFNSTYRV VSVLTVLHQDWLNGKEYKCKVSNKGLPSSIEKTISKAKG QPREPQVYTLPPSQEEMTKNQVSLTCLVKGFYPSDIAVE WESNGQPENNYKTTPPVLDSDGSFFLYSRLTVDKSRWQE GNVFSCSVMHEALHNHYTQKSLSLSLGGGGGSAPTSSST KKTQLQLEHLLLDLQMILNGINNYKNPKLTAMLTAKFYM PKKATELKHLQCLEEELKPLEEVLNLAQSKNFHLRPRDL ISNINVIVLELKGSETTFMCEYADETATIVEFLNRWITF CQSIISTLT | 594 (Monomer) |

(continued)

| SEQ. ID NO: | Component | Sequence | Number of amino acids |
|---|---|---|---|
| 12 | Fusion Protein B | GCCACCATGAAATGGGTCACCTTTATCTCCCTGCTGTTC CTGTTCTCCTCCGCCTACTCCAACTGGGTCAACGTGATC TCCGACCTGAAGAAGATCGAGGACCTGATCCAGTCCATG CACATCGACGCTACCCTGTACACCGAGTCCGACGTGCAC CCTTCCTGTAAAGTGACCGCCATGAAGTGCTTTCTGCTG GAACTGCAAGTGATCTCCCTGGAATCCGGCGACGCCTCT ATCCACGACACCGTGGAAAACCTGATCATCCTGGCCAAC AACTCCCTGTCCTCCAACGGCAACGTGACCGAGTCTGGC TGCAAGAGTGCGAGGAACTGGAAGAGAAGAACATCAAA GAGTTCCTCCAGTCCTTCGTGCACATCGTGCAGATGTTC ATCAACACCTCTGGCGGCGGAGGATCTGGCGGAGGTGGA AGCGGAGGCGGAGGTAGCGGTGGTGGTGGATCTATCACA TGCCCTCCACCTATGTCCGTGGAACACGCCGACATCTGG GTCAAGTCCTACAGCCTGTACTCCAGAGAGCGGTACATC TGCAACTCCGGCTTCAAGAGAAAGGCCGGCACCTCTAGC CTGACCGAGTGCGTGCTGAACAAGGCCACCAATGTGGCC CACTGGACCACACCTAGCCTGAAGTGCATCAGGGACCCC GCTCTGGTTCATCAGAGGCCTGCTCCACCTAGTGGTGGC GGTGGTAGTGGCGGAGGCGGTTCAGGTGGCGGCGGATCT GCTGAGTCTAAGTACGGACCTCCTTGTCCACCTTGTCCT GCTCCAGAAGCTGCTGGCGGCCCTTCCGTGTTTCTGTTC CCTCCAAAGCCTAAGGACACCCTGATGATCTCTCGGACC CCTGAAGTGACCTGCGTGGTGGTCGATGTGTCCCAAGAG GATCCCGAGGTGCAGTTCAATTGGTACGTGGACGGCGTG GAAGTGCACAACGCCAAGACCAAGCCTAGAGAGGAACAG TTCAACTCCACCTACAGAGTGGTGTCCGTGCTGACCGTG CTGCACCAGGATTGGCTGAACGGCAAAGAGTACAAGTGC AAGGTGTCCAACAAGGGCCTGCCTTCCAGCATCGAAAAG ACCATCTCCAAGGCCAAGGGCCAGCCTAGGGAACCCCAG GTTTACACCCTGCCTCCAAGCCAAGAGGAAATGACCAAG AACCAGGTGTCCCTGACCTGCCTGGTCAAGGGCTTCTAC CCTTCCGATATCGCCGTGGAATGGGAGAGCAATGGCCAG CCTGAGAACAACTACAAGACAACCCCTCCTGTGCTGGAC TCCGACGGCTCCTTCTTTCTGTACTCCCGCCTGACCGTG GACAAGTCCAGATGGCAAGAGGGCAACGTGTTCTCCTGC TCCGTGATGCACGAGGCCCTGCACAATCACTACACCCAG AAGTCCCTGTCTCTGTCCCTTGGAGGTGGTGGTGGCTCT GCTCCTACCTCCTCCAGCACCAAGAAAACCCAGCTGCAG TTGGAGCATCTGCTGCTGGACCTCCAGATGATCCTGAAT GGCATCAACAATTACAAGAACCCCAAGCTCACCGCCATG CTGACCGCCAAGTTCTACATGCCCAAGAAGGCCACCGAG CTGAAGCACCTCCAGTGCCTGGAAGAGGAACTGAAGCCC CTGGAAGAAGTGCTGAATCTGGCCCAGTCCAAGAACTTC CACCTGAGGCCTCGCGACCTGATCTCCAACATCAATGTG ATCGTGTTGGAGCTGAAGGGCTCCGAGACAACCTTCATG TGCGAGTACGCCGACGAGACAGCTACCATCGTGGAATTT CTGAACCGGTGGATCACCTTCTGCCAGTCCATCATCTCC ACACTGACCTAATAACTCGAG | |

In another aspect, the present invention is directed to a polynucleotide encoding a fusion protein comprising an IL15 protein, an IL15 receptor alpha (IL15Rα) protein, an Fc domain and an IL2 protein, a vector comprising the polynucleotide, and a transformed cell introduced with the vector.

When the nucleotide sequence is prepared by chemical synthesis, a synthesis method well known in the art, for example, a method described in the literature (Engels and Uhlmann, Angew Chem IntEd Engl, 37:73-127, 1988), methods employing triesters, phosphites, phosphoramidite and H-phosphate, PCR and other autoprimer methods, methods of synthesizing oligonucleotide on a solid support, and the like may be used.

In the present invention, the polynucleotide may comprise a nucleic acid sequence represented by SEQ ID NO: 11 or SEQ ID NO: 12, but is not limited thereto.

Meanwhile, the polynucleotide may further comprise a signal sequence or a leader sequence. As used herein, the term "signal sequence" refers to a nucleic acid encoding a signal peptide that directs secretion of a target protein. The signal peptide is cleaved after translation in the host cell. Specifically, the signal sequence of the present invention is a nucleotide encoding an amino acid sequence that initiates the movement of a protein through the ER (endoplasmic reticulum) membrane. Signal sequences useful for the present invention comprise antibody light-chain signal sequences, such as antibody 1418 (Gillies et al., J. Immunol. Meth. 1989 125:191-202), antibody heavy-chain signal sequences, such as MOPC141 antibody heavy-chain signal sequences (Sakano et al., Nature, 1980 286: 676-683) and other signal sequences known in the art (see, for example, Watson et al., Nucleic Acid Research, 1984 12:5145-5164).

In the present invention, the signal peptide may comprise the amino acid sequence represented by SEQ ID NO: 13.

In the present invention, the vector can be introduced into a host cell, recombined therewith, and thus inserted into the host cell genome. Alternatively, the vector is considered a nucleic acid means comprising a polynucleotide sequence capable of spontaneously conducting replication as an episome. The vector includes linear nucleic acids, plasmids, phagemids, cosmids, RNA vectors, viral vectors, and analogs thereof. Examples of the viral vector include, but are not limited to, retroviruses, adenoviruses, and adenoassociated viruses.

Specifically, the vector may be plasmid DNA, phage DNA, or the like, and examples thereof include commercially developed plasmids (pUC18, pBAD, pIDTSAMRT-AMP, etc.), *Escherichia* coli-derived plasmids (pYG601BR322, pBR325, pUC118, pUC119, and the like), *Bacillus subtilis-derived* plasmids (pUB110, pTP5, and the like), yeast-derived plasmids (YEp13, YEp24, YCp50, etc.), phage DNA (Charon4A, Charon21A, EMBL3, EMBL4, λgt10, λgt11, λZAP, and the like), animal virus vectors (retroviruses, adenovirus, vaccinia virus and the like), and insect virus vectors (baculovirus, and the like). The vector exhibits different protein expression levels and modifications depending on the host cell, so it is preferable to select and use host cells that are optimal for the application.

As used herein, the term "gene expression" or "expression" of a protein of interest is intended to mean transcription of a DNA sequence, translation of an mRNA transcript, or secretion of a fusion protein product or a fragment thereof. A useful expression vector may be RcCMV (Invitrogen, Carlsbad) or a variant thereof. The expression vector includes a human cytomegalovirus (CMV) promoter to promote continuous transcription of the target gene in mammalian cells and a bovine growth hormone polyadenylation signal sequence to increase the stable-state level of RNA after transcription.

In the present invention, the host cells of the transformed cells may include, but are not limited to, prokaryotic, eukaryotic, mammalian, plant, insect, fungal, or cellular cells. The prokaryotic cells may, for example, be E. *coli.* In addition, the eukaryotic cells may, for example, be yeast. In addition, examples of mammalian cells include, but are not limited to, CHO cells, F2N cells, CSO cells, BHK cells, Bowes melanoma cells, HeLa cells, 911 cells, AT1080 cells, A549 cells, HEK 293 cells, and HEK293T cells. Any cells may be used so long as they are useful as mammalian host cells known to those skilled in the art.

In addition, when introducing an expression vector into host cells, CaCl$_2$ precipitation, a Hanahan method that applies a reducing substance called DMSO (dimethyl sulfoxide) to CaCl$_2$ precipitation to improve efficiency, electroporation, calcium phosphate precipitation, protoplast fusion, agitation using silicon carbide fibers, agrobacterium-mediated transformation, transformation using PEG, dextran sulfate, and lipofectamine, and drying/inhibition-mediated transformation may be used.

As described above, for optimization of the properties of the fusion protein or for other purposes, the glycan patterns (e.g., sialyation, fucosylation, and glycosylation) of the fusion proteins can be manipulated through manipulation of the glycosylation-related genes possessed by the host cells through a method known to those skilled in the art.

In another aspect, the present invention is directed to a method for producing a fusion protein comprising an IL15 protein, an IL15 receptor alpha (IL15Rα) protein, an Fc domain, and an IL2 protein, wherein the method comprising culturing the transformed cells.

Specifically, the producing method comprises i) culturing the transformed cells to obtain a culture, and ii) recovering a fusion protein from the culture.

The method of culturing the transformed cells may be performed using a method widely known in the art, and specifically, the culture may be continuously carried out by a batch process or an injection-batch or repeated fed-batch process, but is not limited thereto.

In another aspect, the present invention is directed to a fusion protein dimer in which two fusion proteins comprising the fusion protein are bound to each other.

In the present invention, the bond between the fusion proteins constituting the fusion protein dimer may be formed by a disulfide bond (S-S) based on cysteine (C) present in the linker, but is not limited thereto.

The two fusion proteins constituting the dimer may be the same as or different from each other. Preferably, the fusion protein dimer may be a homodimer, but is not limited thereto.

In the present invention, the fusion protein dimer may be prepared by binding monomer fusion proteins prepared by the method for producing fusion proteins through a disulfide bond, or may be prepared using the same method as the method for producing the fusion protein using a vector comprising a polynucleotide encoding the fusion protein dimer or a transformed cell introduced with the vector.

In another aspect, the present invention is directed to a composition for culturing natural killer cells (NK cells) comprising the fusion protein or the fusion protein dimer, and a method of culturing NK cells (natural killer cells) comprising culturing NK cells in a medium containing the composition for culturing NK cells.

In the present invention, the NK cells may be derived from, for example, mammals, humans, monkeys, pigs, horses, cattle, sheep, dogs, cats, mice, or rabbits. The NK cells may be obtained from a normal subject or a cancer patient. The NK cells may be isolated from blood or PBMCs.

In the present invention, the culture may aim to proliferate or activate NK cells. "Proliferation" of the NK cells means an increase in the number of cells, and may be used interchangeably with "growth". The activation of the NK cells means that the NK cells perform their own functions, and is characterized in that the surface antigens of CD16, DNAM1, NKG2C, NKG2D, NKp46, CCR5, or a combination thereof are highly expressed. Activation of the NK cells also means that NK cells perform their own functions, and is characterized in that surface antigens of NKG2A, TIGIT, CD25, CD57, CD96, or a combination thereof are underexpressed.

The surface antigen properties can also be referred to as "immunological properties", and can be detected by observing cell-surface markers (e.g., staining cells with tissue-specific or cell-marker-specific antibodies) using techniques such as flow cytometry or immunocytochemistry, by observing cell surface markers using light microscopy or confocal microscopy, or by measuring changes in gene expression using techniques well known in the art such as polymerase chain reaction (PCR) or gene expression profiling.

"Positive" or "+" may mean that a cell marker is present in a higher amount or at a higher concentration than in other cells acting as a control for the cell marker. Because a marker is present inside or on the surface of cells, if the cells can be distinguished from one or more other cells using the marker, the cells are said to be positive to the marker. In addition, this may mean that the cell has the marker in an amount sufficient to generate a signal, e.g., a signal from a cytometric device, at a value greater than the background value. For example, if cells are detectably labeled with an antibody specific for CD56, and the signal from this antibody is detectably greater than a control (e.g., background value), the cell is said to be "positive for CD56" or "CD56+". The term "negative" or "-" may mean that that even if an antibody specific for a particular cell surface marker is used, the marker cannot be detected compared to the background value. For example, if cells cannot be detectably labeled with an antibody specific for CD3, the cells are said to be "negative for CD3" or "CD3-".

In the present invention, the composition for culturing NK cells may further comprise any one selected from the group consisting of a medium, serum, supplement, and combinations thereof.

The medium for culturing NK cells may contain amino acids, sugars, inorganic salts, or vitamins. Preferably, the medium for culturing NK cells may contain all of amino acids, sugars, inorganic salts, and vitamins.

As used herein, the term "medium for culturing cells" refers to a medium used for culturing cells, and specifically refers to a medium for culturing NK cells, more specifically CD3- CD56+ cells. The medium for culturing cells contains components required by cells for cell growth and survival *in vitro,* or contains components that support cell growth and survival. Specifically, the components may be vitamins, essential or nonessential amino acids, or trace elements. The medium may be a medium used for culturing cells, preferably eukaryotic cells, more preferably NK cells.

In the present invention, the amino acid components comprise at least one amino acid selected from the group consisting of glycine, L-alanine, L-valine, L-leucine, L-isoleucine, L-threonine, L-serine, L-cysteine, L-methionine, L-aspartic acid, L-asparagine, L-glutamic acid, L-glutamine, L-lysine, L-arginine, L-histidine, L-phenylalanine, L-tyrosine, L-tryptophan, L-proline, β-alanine, γ-aminobutyric acid, ornithine, citrulline, homoserine, triiodotyrosine, thyroxine, and deoxyphenylalanine, or a combination thereof.

The vitamin component may include at least one vitamin selected from the group consisting of biotin, D-calcium pantothenate, folic acid, niacinamide, pyridoxine hydrochloride, riboflavin, thiamine hydrochloride, vitamin B12, choline chloride, i-inositol, and ascorbic acid, or a combination thereof, and may preferably include at least one vitamin selected from the group consisting of i-inositol, thiamine hydrochloride, niacinamide, and pyridoxine hydrochloride, or a combination thereof.

The inorganic salt component may include at least one selected from the group consisting of (anhydrous) calcium chloride ($CaCl_2$), copper sulfate pentahydrate ($CuSO_4\text{-}5H_2O$), ferric sulfate heptahydrate ($FeSO_4\text{-}7H_2O$), (anhydrous) magnesium chloride, (anhydrous) magnesium sulfate ($MgSO_4$), potassium chloride (KCl), sodium chloride (NaCl), disodium hydrogen phosphate ($Na_2HPO_4$), sodium hydrogen phosphate monohydrate ($NaH_2PO_4\text{-}H_2O$), zinc sulfate heptahydrate ($ZnSO_4\text{-}7H_2O$), ferric nitrate nonahydrate ($Fe(NO_3)_3\cdot9H_2O$), and sodium bicarbonate ($NaHCO_3$), or a combination thereof.

The other components include at least one selected from the group consisting of D-glucose (dextrose), sodium pyruvate, hypoxanthine Na, thymidine, linoleic acid, lipoic acid, adenosine, cytidine, guanosine, uridine, 2'-deoxyadenosine, 2'-deoxycytidine HCl, and 2'-deoxyguanosine, or a combination thereof.

In the present invention, the composition for culturing cells may further comprise an antibody for activating natural killer cells. The antibody for activating NK cells may be an anti-CD3 antibody, an anti-CD2 antibody, an anti-CD335 antibody, or a combination thereof, but is not particularly limited thereto. The antibody may also comprise beads to which the antibody for activating natural killer cells is bound. In addition, a fusion protein comprising two or more types of the antibody for activating natural killer cells or a variable domain fragment may be used.

In the present invention, the medium may be a medium for culturing animal cells such as DMEM (Dulbecco's modified Eagle's medium), EDM (endothelial differentiation medium), MEM (minimal essential medium), BME (basal medium Eagle), RPMI 1640, F-10, F-12, a-MEM (a-minimal essential medium), G-MEM (Glasgow's minimal essential medium), Iscove's Modified Dulbecco's medium, AIM-V medium, X-vivo™ 15 medium or NK MACS medium.

As used herein, the term "serum" refers to a transparent supernatant isolated from completely coagulated blood. In addition, serum should be added to a synthetic medium for culturing animal cells, and generally used serum is bovine, equine, or human serum. A bovine-derived serum may be fetal bovine serum (FBS), newborn bovine serum, calf serum, adult bovine serum, or the like, depending on the blood collection time. Human-derived serum may be human serum from a donor having a blood type of AB, or human AB serum that has an antibody to antigens of A and B and thus is capable of minimizing immune reactivity. In addition, an alternative to serum such as CTS immune cell SR may be used.

As used herein, the term "supplement" may be GlutaMAX (Gibco®), which is an alternative to L-glutamine, in order to improve stability and cell activity during cell culture. In addition, the supplement may be an NK MACS supplement (Miltenyi Biotec, 130-113-102).

In the present invention, the composition for culturing NK cells may comprise the fusion protein or fusion protein dimer at a concentration of 1 nM to 500 nM. Specifically, the composition for culturing NK cells may comprise the fusion protein or fusion protein dimer at a concentration of 1 nM to 500 nM, preferably 1 nM to 300 nM, more preferably 1 nM to 150 nM, but is not limited thereto. In one embodiment of the present invention, the composition may comprise the fusion protein dimer at a concentration of 1 nM or 50 nM.

In the present invention, the method for culturing NK cells may comprise

(a) isolating cells not expressing CD3 from peripheral blood mononuclear cells (PBMCs), (b) isolating cells expressing CD56 from the cells isolated in step (a), and (c) culturing the cells isolated in step (b) in the presence of the composition comprising the fusion protein or fusion protein dimer.

In the present invention, the culturing method may further comprise obtaining PBMCs from the peripheral blood before the culturing. The culturing the cells in the presence of the composition comprising the fusion protein or fusion protein dimer may comprise isolating NK cells not expressing CD3 (CD3- NK cells) from the obtained PBMCs and isolating NK cells expressing CD56 (CD56+ NK cells) from the isolated NK cells.

In the present invention, the culture may be performed using a method widely known in the art. Specifically, culturing the separated cells may be performed at a culture temperature of 27°C to 40°C, preferably 30°C to 37°C. In one embodiment of the present invention, the culturing may be performed at a temperature of 37°C. In addition, the isolated cells may be cultured in the presence of 1% to 10% $CO_2$, preferably 5% $CO_2$.

In another aspect, the present invention is directed to a composition for preventing or treating cancer, an immune disease, or an infectious disease, wherein the composition comprises the NK cells prepared by the method for culturing NK cells.

In another aspect, the present invention is directed to a method for preventing or treating cancer, an immune disease, or an infectious disease, wherein the method comprises administering the NK cells prepared by the culturing method.

In another aspect, the present invention is directed to the use of the NK cells for the prevention or treatment of cancer, an immune disease, or an infectious disease.

In another aspect, the present invention is directed to the use of the NK cells for preparation of a therapeutic agent for preventing or treating cancer, an immune disease, or an infectious disease.

In the present invention, the term "cancer" is used in the same meaning as "tumor", and the composition for prevention or treatment according to the present invention can be applied to all types of cancer including solid cancer and blood cancer. Solid cancer refers to cancer taking the form of a lump in an organ, unlike blood cancer, and includes most cancer occurring in organs. There is no particular limitation as to the type of cancer that can be treated using the fusion protein and NK cells according to the present invention, and examples of such cancer include, but are not limited to, gastric cancer, liver cancer, lung cancer, colorectal cancer, breast cancer, prostate cancer, ovarian cancer, pancreatic cancer, cervical cancer, thyroid cancer, laryngeal cancer, acute myeloid cancer leukemia, brain tumors, neuroblastoma, retinoblastoma, head and neck cancer, salivary gland cancer, lymphoma, and the like.

As used herein, the term "immune disease" refers to a disease caused by an abnormal immune response, and is, for example, an autoimmune disease, transplant rejection, or arthritis, but is not limited thereto. Examples of the autoimmune disease include, but are not limited to, Crohn's disease, erythematosus, atopy, rheumatoid arthritis, Hashimoto's thyroiditis, pernicious anemia, Addison's disease, type 1 diabetes, lupus, chronic fatigue syndrome, fibromyalgia, hypothyroidism and hyperthyroidism, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma, and ulcerative colitis. The transplant rejection may be allograft rejection or graft-versus-host disease (GVHD).

As used herein, the term "infectious disease" refers to a disease caused by infection with a virus or pathogen, and encompasses all diseases that can be transmitted and infected through respiration, blood transmission, skin contact, or the like. Non-limiting examples of the infectious disease include, but are not limited to, hepatitis B and C, human papilloma virus (HPV) infection, cytomegalovirus infection, viral respiratory disease, influenza, and the like.

The composition for prevention or treatment according to the present invention may comprise a pharmaceutically effective amount of the NK cells prepared by the culture method alone, or may comprise the same in combination with at least one pharmaceutically acceptable carrier, excipient or diluent. The pharmaceutically effective amount refers to an amount sufficient to prevent, ameliorate, or treat symptoms of cancer, an immune disease, or an infectious disease.

As used herein, the term "pharmaceutically acceptable" means that the composition is physiologically acceptable and does not usually cause allergic reactions such as gastrointestinal disorders, dizziness, or similar reactions when administered to humans. Examples of the carrier, excipient or diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oils. In addition, the composition may further comprise fillers, anti-aggregating agents, lubricants, wetting agents, flavors, emulsifiers and preservatives.

In addition, the composition of the present invention may comprise at least one known active ingredient having an effect of treating cancer, an immune disease, or an infectious disease, along with the NK cells produced by the culture method.

The composition of the present invention may be formulated using a method known in the art to provide rapid, sustained or delayed release of the active ingredient after administration to a mammal other than human. The formulation may be in the form of a powder, granule, tablet, emulsion, syrup, aerosol, soft or hard gelatin capsule, sterile injectable solution, or sterile powder.

The composition of the present invention may be administered through various routes, including oral, transdermal, subcutaneous, intravenous, or intramuscular administration, and the dosage of the active ingredient depends on various factors, such as the route of administration, the patient's age, gender, and weight, and the severity of disease of the patient. The composition for preventing or treating cancer, an immune disease, or an infectious disease according to the present invention may be administered in combination with a known compound having an effect of preventing, ameliorating or treating symptoms of cancer, an immune disease, or an infectious disease.

Hereinafter, the present invention will be described in more detail with reference to examples. However, it will be obvious to those skilled in the art that these examples are provided only for illustration of the present invention and should not be construed as limiting the scope of the present invention.

**Example 1: Preparation and culture of CD3- CD56+ NK cells**

Example 1-1: Preparation of CD3- CD56+ NK cells

**(1) STEP 1: Isolation of PBMC from LRS chamber**

1) An LRS chamber was purchased from the Korean Red Cross Nambu Blood Center.
2) A main ingredient was recovered from the LRS chamber using 30 mL of cliniMACS buffer.
3) 30 mL of a dilution of the main ingredient was overlaid on 15 mL of Ficoll-Paque medium.
4) A tube containing a dilution of the main ingredient was washed with CliniMACS buffer and was overlaid on 15 mL of Ficoll-Paque medium.
5) The result was broken off by centrifugation at 400×g and at room temperature for 30 minutes.
6) The entire supernatant including the PBMC layer was recovered.

**(2) STEP 2: Removal of CD3 from PBMCs**

1) The number of PBMC cells recovered in STEP 1 was counted.

2) The cell suspension was centrifuged at 340×g for 10 minutes. The supernatant was completely removed.

3) A cell pellet was resuspended in 80 μL of CliniMACS buffer (a solution containing 0.5% human serum albumin (HSA) and 2 mM EDTA at a pH of 7.2) per $10^7$ cells.

4) 20 μL of CD3 MicroBeads (Miltenyi Biotec, Cat#130-050-101) per $10^7$ cells was added thereto.

5) The result was thoroughly mixed and incubated in a refrigerator (4°C) for 15 minutes.

6) The cells were washed with 1 to 2 mL of buffer per $10^7$ cells and centrifuged at 340×g for 10 minutes. The supernatant was completely removed.

7) The cell pellet was resuspended in 500 μL buffer per $1×10^8$ cells.

8) A column was mounted on a MACs separator and rinsed with 2 mL of buffer.

9) The resuspended cells were loaded on the column.

10) Unlabeled cells that passed through the column were harvested, and the column was washed with 3×2 mL of buffer. The total effluent was collected.

**(3) STEP 3: Enrichment of CD56$^+$ cells**

1) The number of cells obtained in STEP 2 was counted.

2) The cell suspension was centrifuged at 340×g for 10 minutes. The supernatant was completely removed.

3) A cell pellet was resuspended in 80 μL of CliniMACS buffer per a total of $10^7$ cells.

4) 20 μL of CD56 MicroBeads (Miltenyi Biotec, Cat#130-050-401) per a total of $10^7$ cells was added thereto.

5) The result was thoroughly mixed and incubated in a refrigerator (4°C) for 15 minutes.

6) The cells were washed with 1 to 2 mL of buffer per $10^7$ cells, and centrifuged at 340×g for 10 minutes. The supernatant was completely removed.

7) The cell pellet was resuspended in 500 μL buffer per $1×10^8$ cells.

8) A column was mounted on a MACs separator, and was rinsed with 3 mL of buffer.

9) The resuspended cells were loaded into the column.

10) Cells that passed through the column were further loaded three times.

11) The column was removed from the separator and placed in a 15 ml collection tube.

12) 5 mL of buffer was added to the column.

13) A plunger was strongly pushed into the column to recover magnetically labeled cells.

The total cell number, viability, and isolation yield measured in each step are shown in Table 3 below.

[Table 3]

| | Total Cell number (X10$^6$) | Viability (%) | Yield (%) |
|---|---|---|---|
| PBMC | 256.8 | 98.305 | N/A |
| CD3$^-$ | 172.0 | 99.13 | 67.0 |
| CD3$^-$ CD56$^+$ | 44.4 | 98.76 | 17.3 |

The results of D0 FACS analysis of isolated NK cells obtained as a result of STEP 3 are shown in FIG. 2.

Example 1-2: Preparation of culture composition and culture of CD3- CD56+ NK cells

The basic components of the medium for culturing CD3-CD56+ NK cells are shown in Table 4 below, and the additives for each experimental group are shown in Table 5.

**(1) Preparation of culture composition**

[Table 4]

| Component | | | | Volume | Final Concentration |
|---|---|---|---|---|---|
| Products | | Manufactu rer | Cat. # | | |
| Basic components of medium | Human AB serum | Sigma | H4522-100 mL | 2.5 ml | 5% |
| | NK MACS supplement | Miltenyi Biotec | 130-113-102 | 500 μL | 1% |
| | NK MACS medium | Miltenyi Biotec | 130-112-968 | to 50 ml | |

[Table 5]

| Experimental group | Fusion protein for proliferating NK cells | | Beads for stimulating NK cells | |
|---|---|---|---|---|
| Comparative Example 1 | Fc-IL2WT | 1 nM | - | |
| Comparative Example 2 | IL15R-IL15-Fc + Fc-IL2v | 1 nM | - | |
| Comparative Example 3 | IL15R-IL15-Fc + Fc-IL2wt | 1 nM | - | |
| Experimental Example 1-1 | Fusion protein A | 1 nM | - | |
| Experimental Example 1-2 | | 50 nM | NK Activation/ Expansion Beads (CD2/CD335) | 5 μL/ $1 \times 10^6$ NK cells |
| Experimental Example 1-3 | | 50 nM | - | |
| Experimental Example 2-1 | Fusion protein B | 1 nM | - | |
| Experimental Example 2-2 | | 50 nM | NK Activation/ Expansion Beads (CD2/CD335) | 5 μL/ $1 \times 10^6$ NK cells |
| Experimental Example 2-3 | | 50 nM | - | |

**(2) Culture of NK cells**

The NK cells (CD3-/CD56+) isolated in Example 1-1 were seeded at $1 \times 10^6$ cells/mL in a flask or well plate. A medium composition containing the basic medium components and additives shown in Tables 4 and 5 was dispensed in each well in which the cells were seeded and incubated at 37°C in the presence of 5% $CO_2$. However, the beads for stimulating NK cells in Table 5 (Miltenyi Biotec, Cat# 130-094-483) were added only at the time of initial seeding. The number of cells was counted every 2 days from the 6th day of seeding, and the cells were sub-cultured in the medium compositions for respective experimental groups sequentially added to a 48-well plate, a 24-well plate, a 12-well plate, a 6-well plate, a 25T flask and a 75T flask when the confluency of the cells reached 80% or more of a culture vessel, and all cells were finally harvested on the 18th day.

The total cell number and viability of the natural killer cells cultured on days 6, 8, 10, 12, 14, 16, and 18 were compared, and the results are shown in Tables 6 and 7 below.

[Table 6]

| Experimental group | Total cell number | | | | | | | | Fold |
|---|---|---|---|---|---|---|---|---|---|
| | Day 0 | Day 6 | Day 8 | Day 10 | Day 12 | Day 14 | Day 16 | Day 18 | |
| Comparative Example 1 | 1.00 | 0.70 | 0.88 | 1.47 | 1.75 | 1.40 | 1.67 | 1.23 | 26.37 |
| Comparative Example 2 | 1.00 | 0.83 | 0.89 | 1.38 | 1.47 | 1.29 | 1.63 | 1.05 | 35.72 |
| Comparative Example 3 | 1.00 | 0.78 | 0.96 | 1.49 | 1.50 | 1.07 | 1.39 | 0.95 | 49.34 |
| Experimental Example 1-1 | 1.00 | 0.78 | 0.99 | 1.57 | 2.03 | 1.56 | 1.79 | 1.48 | 79.75 |
| Experimental Example 1-2 | 1.00 | 3.17 | 2.66 | 3.11 | 2.50 | 1.88 | 1.29 | 1.69 | 903.69 |
| Experimental Example 1-3 | 1.00 | 1.36 | 1.99 | 2.16 | 2.22 | 1.50 | 2.04 | 1.21 | 289.86 |
| Experimental Example 2-1 | 1.00 | 0.72 | 0.92 | 1.77 | 2.55 | 1.83 | 1.72 | 1.55 | 74.52 |
| Experimental Example 2-2 | 1.00 | 2.92 | 2.80 | 3.26 | 2.23 | 2.08 | 2.00 | 2.01 | 2519.34 |
| Experimental Example 2-3 | 1.00 | 1.38 | 1.58 | 2.77 | 1.72 | 2.08 | 1.40 | 1.44 | 172.24 |

[Table 7]

| Experimental group | Viability | | | | | | | |
|---|---|---|---|---|---|---|---|---|
| | Day 0 | Day 6 | Day 8 | Day 10 | Day 12 | Day 14 | Day 16 | Day 18 |
| Comparative Example 1 | 98.76 | 95.40 | 95.51 | 96.59 | 94.86 | 94.83 | 95.90 | 91.08 |
| Comparative Example 2 | 98.76 | 95.68 | 95.51 | 96.87 | 95.23 | 94.41 | 96.13 | 95.88 |
| Comparative Example 3 | 98.76 | 95.73 | 95.83 | 97.43 | 94.50 | 95.88 | 96.10 | 90.05 |
| Experimental Example 1-1 | 98.76 | 92.54 | 94.83 | 97.00 | 97.25 | 96.75 | 96.31 | 92.22 |
| Experimental Example 1-2 | 98.76 | 94.85 | 95.78 | 97.49 | 96.46 | 97.83 | 97.15 | 96.90 |
| Experimental Example 1-3 | 98.76 | 94.03 | 96.25 | 97.51 | 97.63 | 98.22 | 97.99 | 96.70 |
| Experimental Example 2-1 | 98.76 | 92.06 | 93.96 | 95.05 | 94.89 | 93.38 | 95.22 | 90.04 |
| Experimental Example 2-2 | 98.76 | 95.27 | 96.37 | 97.61 | 97.53 | 98.52 | 98.21 | 96.31 |
| Experimental Example 2-3 | 98.76 | 92.40 | 96.44 | 97.88 | 96.70 | 97.95 | 97.97 | 96.69 |

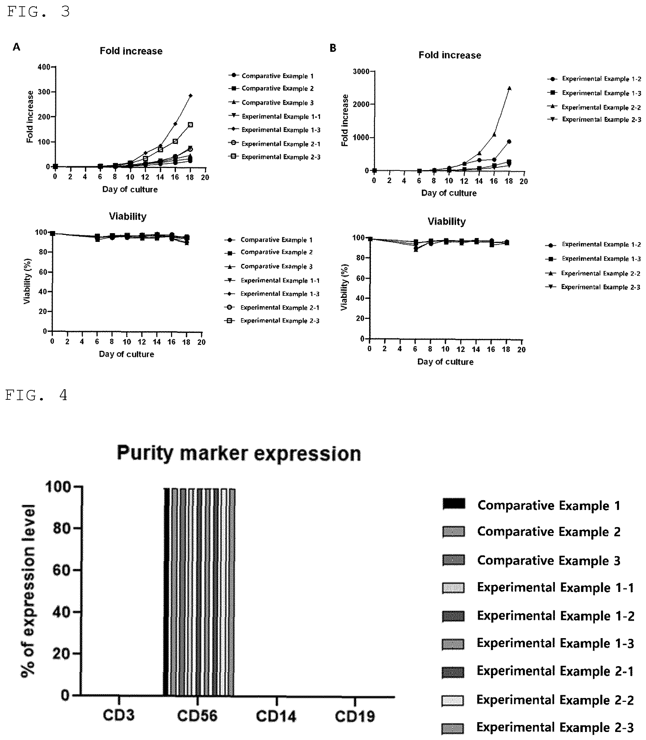

The result showed that, compared to culturing using Comparative Examples 1, 2, 3, the proliferation rate and survival rate of natural killer cells were significantly increased, regardless of the concentration of the treated material when culturing using fusion protein A (Experimental Examples 1-1, 1-2, and 1-3) and fusion protein B (Experimental Examples 2-1, 2-2, and 2-3) (FIG. 3A).

When cultured using a fusion protein (Examples 1-3 and 2-3) at a concentration of 50 nM compared to culture using a fusion protein at a concentration of 1 nM (Experimental Examples 1-1 and 2-1), the proliferation rate and survival rate of natural killer cells were increased.

In addition, culturing using beads for stimulating NK cells (Experimental Examples 1-3 and 2-3) exhibited a greater effect on cell proliferation compared to culturing without using beads (Experimental Examples 1-2 and 2-2) (FIG. 3B).

**Example 2: Identification of expression of activation markers in cultured NK cells**

Example 2-1: Surface staining of CD3- CD56+ NK cells

1) PBMCs or CD3- CD56+ cells were prepared at a final concentration of 1 to $2.5 \times 10^6$ cells/mL.
2) 200 $\mu$L aliquots of the cell suspension (2 to $5 \times 10^5$ cells) were dispensed into the round-bottom wells of the plate.
3) The cells were pelleted by centrifugation at $340 \times g$ and 4°C for 5 minutes.

4) The supernatant was removed and the cells were washed in 200 μL of cold FACS Buffer.

5) The cells were pelleted by centrifugation at 340×g and 4°C for 5 minutes, and the supernatant was removed.

6) In order to block non-specific Fc-mediated interactions, 50 μL of a diluted Fc Block (200:1) was added to each well, followed by incubation at 4°C for 10 minutes.

7) The antibodies against antigens shown in Table 8 below and a fluorescence minus one (FMO) control antibody were added to the cells, followed by incubation on ice in the absence of light for 20 minutes.

8) The cells were washed twice with 200 μL FACS buffer. The cells were centrifuged at 340×g for 5 min.

9) The supernatants were carefully removed from the cell pellets.

10) The plate was gently tapped to release the cell pellets.

11) The cell pellets were resuspended in 200 μL of fixation buffer.

12) The stained cell samples were analyzed by flow cytometry.

[Table 8]

| Antigen | Clone | Producer | Cat# |
|---|---|---|---|
| CD3 | HIT3a | BioLegend | 300326 |
| CD56 | B159 | BD Pharmingen | 557747 |
| CD14 | M5E2 | BD Pharmingen | 557923 |
| CD19 | SJ25C1 | BD Horizon | 563325 |
| CD16 | 3G8 | BD Pharmingen | 560195 |
| DNAM-1 | DX11 | BD Pharmingen | 564797 |
| NKG2C | 134591 | BD OptiBuild | 748169 |
| NKG2D | 1D11 | BD Horizon | 562498 |
| NKp46 | 9E2 | BioLegend | 331936 |
| CCR5 | 3A9 | BD Pharmingen | 550856 |
| NKG2A | 131411 | BD OptiBuild | 747923 |
| TIGIT | 741182 | BD OptiBuild | 747840 |
| CD25 | M-A251 | BD Horizon | 563352 |
| CD57 | NK-1 | BD Horizon | 565285 |
| CD96 | 6F9 | BD Pharmingen | 562379 |

Example 2-2: Intracellular staining of CD3- CD56+ NK cells

1) PBMCs or CD3- CD56+ cells were prepared at a final concentration of 1 to $2.5\times10^6$ cells/mL.

2) 200 μL aliquots of the cell suspension (2 to $5\times10^5$ cells) were dispensed into round-bottom wells of the plate.

3) The cells were pelleted by centrifugation at 340×g and 4°C for 5 minutes.

4) In order to prepare a stimulating medium, 500X stimulating cocktail (PMA and ionomycin) was diluted at a concentration of 1X in complete medium (200 μl/well).

5) The supernatant was removed and the cells were resuspended in 200 μL of stimulating medium.

6) The cells were incubated at 37°C in a 5% $CO_2$ incubator for 4 hours.

7) The cells were pelleted by centrifugation at 340×g and 4°C for 5 minutes.

8) The supernatant was removed and the cells were washed with 200 μL cold FACS Buffer.

9) The cells were pelleted by centrifugation at 340×g and 4°C for 5 minutes, and the supernatant was removed.

10) In order to block non-specific Fc-mediated interactions, 50 μL of a diluted Fc block (200:1) was added to each well, followed by incubation at 4°C for 10 minutes.

11) The antibodies against antigens shown in Table 8 above and fluorescence minus one (FMO) control antibody were added to the cells, followed by incubation on ice in the absence of light for 20 minutes for surface staining.

12) The cells were washed twice with 200 μL FACS buffer. The cells were centrifuged at 340×g for 5 min.

13) The supernatants were carefully removed from the cell pellets.

14) The plate was gently tapped to release the cell pellets.

15) The respective cell pellets were resuspended in 100μL CytoFix/CytoPerm solution.

16) The cells were incubated in a refrigerator or on ice in the absence of light for 30 min.

17) The cells were washed twice with 200 μL of 1X Perm/Wash Buffer.

18) For intracellular staining, the antibodies to antigens shown in Table 9 and the fluorescence minus one (FMO) control antibody were added to the cells, followed by incubation on ice in the absence of light for 20 minutes.

19) The cells were washed with 200 μL perm/wash buffer. The cells were centrifuged at 340xg for 5 minutes.

20) The supernatants were carefully sucked out from the cell pellets.

21) The plate was gently tapped to release the cell pellets.

22) The cell pellets were resuspended with 200 μL fixation buffer.

23) The stained cell samples were analyzed by flow cytometry.

[Table 9]

| Antigen | Clone | Producer | Cat# |
|---------|-------|----------|------|
| IFN-gamma | 4S.B3 | BioLegend | 502506 |
| Granzyme B | QA16A02 | BioLegend | 372214 |
| Perforin | B-D48 | BioLegend | 353312 |

Example 2-3: Cell Characterization (Flow cytometry)

The natural killer cells (CD3-CD56+) cultured in the culture composition of Example 1-2 were cultured for 18 days, and then the purity and expression of activation markers and inhibition markers were identified (Tables 10 to 12 and FIGS. 4 to FIGS. 6).

[Table 10]

| Groups | CD16 | DNAM-1 | NKG2C | NKG2D | NKp4 6 | CCR5 |
|--------|------|--------|-------|-------|--------|------|
| Comparative Example 1 | 26.50 | 98.70 | 2.94 | 88.30 | 96.00 | 44.30 |
| Comparative Example 2 | 27.60 | 98.10 | 4.26 | 89.20 | 96.40 | 49.00 |
| Comparative Example 3 | 29.40 | 98.10 | 4.02 | 89.80 | 96.30 | 47.70 |
| Experimental Example 1-1 | 28.80 | 99.10 | 3.00 | 87.40 | 97.30 | 63.40 |
| Experimental Example 1-2 | 62.40 | 97.30 | 6.11 | 91.90 | 93.20 | 96.80 |
| Experimental Example 1-3 | 33.90 | 99.60 | 1.60 | 81.30 | 96.70 | 73.50 |
| Experimental Example 2-1 | 33.10 | 99.70 | 1.61 | 83.80 | 98.40 | 78.70 |
| Experimental Example 2-2 | 62.40 | 98.50 | 4.64 | 88.40 | 94.60 | 94.70 |
| Experimental Example 2-3 | 34.30 | 99.60 | 1.08 | 68.20 | 96.40 | 58.00 |
| ※ Activation marker Expression level (%) | | | | | | |

[Table 11]

| Groups | NKG2A | TIGIT | CD25 | CD57 | CD96 |
|--------|-------|-------|------|------|------|
| Comparative Example 1 | 10.80 | 14.10 | 0.39 | 11.40 | 91.00 |
| Comparative Example 2 | 17.30 | 10.50 | 0.64 | 15.00 | 89.90 |
| Comparative Example 3 | 16.90 | 15.30 | 0.31 | 12.10 | 90.10 |
| Experimental Example 1-1 | 11.00 | 17.80 | 0.18 | 12.60 | 91.20 |
| Experimental Example 1-2 | 5.58 | 39.50 | 0.65 | 21.00 | 87.60 |

(continued)

| Groups | NKG2A | TIGIT | CD25 | CD57 | CD96 |
|---|---|---|---|---|---|
| Experimental Example 1-3 | 7.94 | 12.80 | 1.26 | 29.20 | 86.10 |
| Experimental Example 2-1 | 6.85 | 13.30 | 0.77 | 24.70 | 82.20 |
| Experimental Example 2-2 | 4.57 | 35.20 | 1. 60 | 21.60 | 82.60 |
| Experimental Example 2-3 | 5.13 | 12.00 | 1.89 | 33.20 | 77.20 |
| ※ Inhibition marker Expression level (%) | | | | | |

[Table 12]

| Groups | PD-1 | Tim-3 | IFN-gamma | Granzyme B | Perforin |
|---|---|---|---|---|---|
| Comparative Example 1 | 0.07 | 0.04 | 49.30 | 98.70 | 95.90 |
| Comparative Example 2 | 0.03 | 0.02 | 56.40 | 97.40 | 96.10 |
| Comparative Example 3 | 0.06 | 0.39 | 58.10 | 98.50 | 96.70 |
| Experimental Example 1-1 | 0.03 | 0.03 | 51.10 | 99.10 | 96.90 |
| Experimental Example 1-2 | 0.04 | 0.02 | 30.30 | 99.70 | 96.00 |
| Experimental Example 1-3 | 0.03 | 0.01 | 38.40 | 99.30 | 95.50 |
| Experimental Example 2-1 | 0.01 | 0.02 | 54.40 | 99.40 | 96.60 |
| Experimental Example 2-2 | 0.02 | 0.00 | 44.30 | 99.70 | 96.60 |
| Experimental Example 2-3 | 0.04 | 0.04 | 73.30 | 98.90 | 96.60 |
| ※ Intracellular marker Expression level (%) | | | | | |

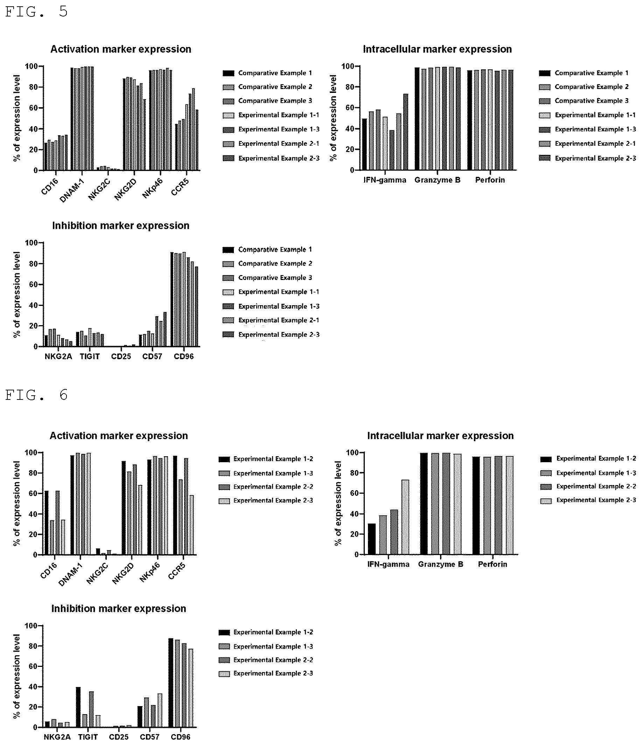

The result showed that the natural killer cells cultured using fusion protein A (Experimental Examples 1-1, 1-2, and 1-3) and fusion protein B (Experimental Examples 2-1, 2-2, and 2-3) exhibited high expression of markers involved in activity and cancer killing and low expression of markers involved in inhibition, comparable to the natural killer cells cultured in Comparative Examples 1, 2 and 3 (FIG. 5).

In addition, culturing using beads for stimulating NK cells (Experimental Examples 1-3 and 2-3) exhibited high expression of CD16, NKG2D, and CCR5 compared to culturing without using beads (Experimental Examples 1-2 and 2-2) (FIG. 6) .

**Example 3: Identification of cancer-cell-killing ability of CD3- CD56+ NK cells**

Example 3-1: Test for determining cancer-cell-killing effect

1) Target cells (cancer cells) were stained with 30 $\mu$l/mL of Calcein-acetoxymethyl (AM) in a $CO_2$ incubator for 1 hour. Cancer cell line information is shown in Table 13 below.

[Table 13]

| Cancer Cell | Origin, Organ | Disease |
|---|---|---|
| K562 | Lymphoma | Chronic Myelogenous Leukemia (CML) |
| BT-474 | Breast | Ductal Carcinoma |
| MDA-MB-231 | Breast | Adenocarcinoma |

2) After incubation, the target cells were washed twice with media.

3) Effector cells (NK cells) were prepared at an appropriate ratio of effector cells to target cells (10:1, 3:1, 1:1).

4) The effector cells were co-cultured in triplicate along with target cell lines in a 96-well plate at 37°C and 5% $CO_2$ at predetermined ratios (10:1, 3:1, 1:1) for 4 hours.

5) In order to measure maximum fluorescence, the stained target cells were treated with Triton-X100, cultured for 4 hours, and then incubated for 4 hours, and spontaneous fluorescence emission therefrom was measured.

6) After 4 hours, the cells were centrifuged at 2,000 rpm and 4°C for 3 minutes, and 100 μL of the supernatant was transferred to a 96-well black plate.

7) Fluorescence was measured with a fluorescence microplate reader at an excitation wavelength of 485 nm and an emission wavelength of 535 nm.

8) The specific Calcein-AM release (%) was calculated according to the following formula:

$$\% \ Specific \ release = ((mean \ experimental \ release-mean \ spontaneous \ release)/(mean \ maximal \ release-mean \ spontaneous \ release)) \times 100$$

Example 3-2: Identification of cancer-cell-killing effect

Natural killer cells (CD3- CD56+) cultured in the culture composition of Example 1-2 were cultured for 18 days, and then the ability thereof to kill K562, BT-474, and MDA-MB-231 cancer cell lines was identified (Tables 14 to 16).

[Table 14]

| Groups | E10 | E3 | E1 |
|---|---|---|---|
| Comparative Example 1 | 85.9 | 72.8 | 38.7 |
| Comparative Example 2 | 85.4 | 69.3 | 35.7 |
| Comparative Example 3 | 84.1 | 70.1 | 40.8 |
| Experimental Example 1-1 | 95.4 | 85.3 | 58.5 |
| Experimental Example 1-2 | 88.0 | 85.7 | 54.5 |
| Experimental Example 1-3 | 93.5 | 82.7 | 46.6 |
| Experimental Example 2-1 | 96.1 | 86.6 | 50.8 |
| Experimental Example 2-2 | 96.6 | 88.3 | 56.2 |
| Experimental Example 2-3 | 90.4 | 64.6 | 30.7 |
| ※ K562 lysis (%) | | | |

[Table 15]

| Groups | E10 | E3 | E1 |
|---|---|---|---|
| Comparative Example 1 | 57.8 | 46.0 | 27.5 |
| Comparative Example 2 | 62.4 | 44.5 | 30.4 |
| Comparative Example 3 | 60.2 | 43.4 | 27.3 |
| Experimental Example 1-1 | 69.8 | 62.4 | 42.7 |
| Experimental Example 1-2 | 76.1 | 67.4 | 44.7 |
| Experimental Example 1-3 | 92.9 | 75.9 | 42.8 |
| Experimental Example 2-1 | 83.9 | 76.3 | 41.0 |
| Experimental Example 2-2 | 86.9 | 76.7 | 43.9 |

(continued)

| Groups | E10 | E3 | E1 |
|---|---|---|---|
| Experimental Example 2-3 | 64.3 | 39.4 | 22.1 |
| ※ BT-474 Lysis (%) | | | |

[Table 16]

| Groups | E10 | E3 | E1 |
|---|---|---|---|
| Comparative Example 1 | 68.3 | 42.5 | 16.8 |
| Comparative Example 2 | 66.3 | 37.9 | 15.1 |
| Comparative Example 3 | 61.7 | 34.5 | 15.7 |
| Experimental Example 1-1 | 69.5 | 41.8 | 21.3 |
| Experimental Example 1-2 | 79.9 | 57.8 | 26.8 |
| Experimental Example 1-3 | 89.8 | 63.9 | 29.2 |
| Experimental Example 2-1 | 82.1 | 52.9 | 22.8 |
| Experimental Example 2-2 | 89.1 | 64.9 | 32.7 |
| Experimental Example 2-3 | 67.8 | 30.2 | 10.8 |
| ※ MDA-MB-231 Lysis (%) | | | |

The result showed that the natural killer cells cultured by treatment with fusion protein A (Experimental Examples 1-1, 1-2, 1-3) and fusion protein B (Experimental Examples 2-1, 2-2, 2-3) in all ratios of effector cells and target cells (E10, E3, E1) exhibited better cancer-cell-killing ability than that of the natural killer cells cultured by treatment with Comparative Examples 1, 2 and 3. In addition, the result showed that culturing using the fusion protein added with beads for stimulating NK cells (Experimental Examples 1-3 and 2-3) exhibited better cancer-cell-killing ability than that of natural killer cells cultured without using beads (Experimental Examples 1-2 and 2-2).

## INDUSTRIAL APPLICABILITY

The fusion protein according to the present invention enables NK cells to be efficiently proliferated and cultured while maintaining the killing ability of NK cells and promoting activation thereof, and thus is capable of yielding a large number of NK cells without using feeder cells, thus being useful for commercialization of cell therapy products with increased safety.

Although specific configurations of the present invention have been described in detail, those skilled in the art will appreciate that this detailed description is provided as preferred embodiments for illustrative purposes only, and should not be construed as limiting the scope of the present invention. Therefore, the substantial scope of the present invention is defined by the accompanying filed claims and equivalents thereto.

**Sequence List Free Text**

An electronic file is attached.

## Claims

1. A fusion protein comprising an IL15 protein, an IL15 receptor alpha (IL15Rα) protein, an Fc domain, and an IL2 protein.

2. The fusion protein according to claim 1, wherein the IL15 protein comprises an amino acid sequence represented by SEQ ID NO: 1.

3. The fusion protein according to claim 1, wherein the IL15 receptor alpha (IL15Rα) protein comprises an amino acid sequence represented by SEQ ID NO: 3.

4. The fusion protein according to claim 1, wherein the Fc domain comprises a heavy-chain constant region 2 (CH2) and a heavy-chain constant region 3 (CH3) of an immunoglobulin.

5. The fusion protein according to claim 4, wherein the immunoglobulin is IgG, IgA, IgE, IgD, or IgM.

6. The fusion protein according to claim 4, wherein the Fc domain comprises an amino acid sequence represented by SEQ ID NO: 5.

7. The fusion protein according to claim 1, wherein the IL2 protein is a wild type or a variant.

8. The fusion protein according to claim 7, wherein the IL2 protein comprises an amino acid sequence represented by SEQ ID NO: 7 or 8.

9. The fusion protein according to claim 1, wherein the fusion protein comprises the following Formula (I):

N'-X-[Linker(1)]l-Y-[Linker(2)]n-Fc domain-[Linker(3)]m-Z-C'          (Formula (I))

Wherein,

N' is an N-terminus of the fusion protein, and C' is a C-terminus of the fusion protein,
X is an IL15 protein, Y is an IL15 receptor alpha (IL15Rα) protein, and Z is an IL2 protein,
Linker (1), Linker (2) and Linker (3) are peptide linkers, and
l, n and m are each independently 0 or 1.

10. The fusion protein according to claim 9, wherein the Linker (1) comprises an amino acid sequence represented by SEQ ID NO: 2.

11. The fusion protein according to claim 9, wherein the Linker (2) comprises an amino acid sequence represented by SEQ ID NO: 4.

12. The fusion protein according to claim 9, wherein the Linker (3) comprises an amino acid sequence represented by SEQ ID NO: 6.

13. The fusion protein according to claim 1, wherein the fusion protein comprises an amino acid sequence represented by SEQ ID NO: 9 or 10.

14. A polynucleotide encoding the fusion protein according to any one of claims 1 to 13.

15. A vector comprising the polynucleotide according to claim 14.

16. A transformed cell introduced with the vector according to claim 15.

17. A method for producing a fusion protein comprising culturing the transformed cell according to claim 16.

18. A fusion protein dimer in which two fusion proteins comprising the fusion protein of claim 1 are bound to each other.

19. The fusion protein dimer according to claim 18, wherein the fusion protein dimer is a homodimer.

20. A composition for culturing natural killer cells (NK cells) comprising the fusion protein according to any one of claims 1 to 13 or the fusion protein dimer according to claim 18.

21. A method of culturing natural killer cells (NK cells) comprising culturing NK cells in a medium which contains the composition according to claim 20.

22. The method according to claim 21, comprising:

(a) isolating cells not expressing CD3 from peripheral blood mononuclear cells (PBMCs);
(b) isolating cells expressing CD56 from the cells isolated in step (a); and
(c) culturing the cells isolated in step (b) in the presence of the composition according to claim 20.

23. A composition for treating cancer, an immune disease, or an infectious disease comprising NK cells prepared by the method according to claim 21.

FIG. 1

FIG. 2

FIG. 3

FIG. 4

FIG. 5

FIG. 6

## INTERNATIONAL SEARCH REPORT

| International application No. |
| --- |
| **PCT/KR2021/011575** |

| | |
| --- | --- |
| **A.** | **CLASSIFICATION OF SUBJECT MATTER** |

**C07K 14/54**(2006.01)i; **C07K 14/715**(2006.01)i; **C07K 14/55**(2006.01)i; **C12N 5/0783**(2010.01)i; **A61K 35/17**(2014.01)i; **A61P 35/00**(2006.01)i; **A61P 31/00**(2006.01)i

According to International Patent Classification (IPC) or to both national classification and IPC

| | |
| --- | --- |
| **B.** | **FIELDS SEARCHED** |

Minimum documentation searched (classification system followed by classification symbols)

C07K 14/54(2006.01); A61K 38/00(2006.01); A61K 38/20(2006.01); C07K 14/55(2006.01); C07K 14/715(2006.01); C07K 14/735(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: IL-15 단백질(interleukin 15 protein), IL-15 수용체 알파 단백질(interleukin 15 receptor alpha protein), Fc 도메인(Fc domain), IL-2 단백질(interleukin 2 protein), 융합단백질(fusion protein), NK 세포(NK cell), 배양(culture)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X<br>Y<br>A | WU, Z. et al. IL-15Rα-IgG1-Fc enhances IL-2 and IL-15 anti-tumor action through NK and CD8+ T cells proliferation and activation. Journal of Molecular Cell Biology. 2010, vol. 2, pp. 217-222.<br>See abstract, pages 218-221, and figures 1(A) and 2-4. | 1,14-17,20-23<br>2-12,18,19<br>13 |
| Y | WO 2019-246379 A1 (CUGENE INC.) 26 December 2019 (2019-12-26)<br>See abstract, claims 1, 4, 8-9 and 13-14, SEQ ID NOs: 2, 5 and 11, and figure 1E. | 2-3,9-12 |
| Y | KR 10-2020-0032009 A (GI INNOVATION, INC.) 25 March 2020 (2020-03-25)<br>See claims 1-3, 12-14,16-18, 21-23 and 25-26, paragraphs [0025], [0059] and [0095]-[0096], and SEQ ID NOs: 3-5 and 10. | 4-12,18,19 |
| A | WO 2019-213517 A1 (IMMUNE TARGETING INC.) 07 November 2019 (2019-11-07)<br>See entire document. | 1-23 |

| | |
| --- | --- |
| ✓ Further documents are listed in the continuation of Box C. | ✓ See patent family annex. |

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **08 December 2021** | **08 December 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/011575** |

| C. | DOCUMENTS CONSIDERED TO BE RELEVANT | |
| --- | --- | --- |
| Category* | Citation of document, with indication, where appropriate, of the relevant passages | Relevant to claim No. |
| A | ZHENG, X. X. et al. IL-2 receptor-targeted cytolytic IL-2/Fc fusion protein tratment blocks diabetogenic autoimmunity in nonobese diabetic mice. The Journal of Immunology. 1999, vol. 163, pp. 4041-4048.<br>See entire document. | 1-23 |
| A | WO 2005-014642 A2 (TRANSGENE S.A.) 17 February 2005 (2005-02-17)<br>See entire document. | 1-23 |

Form PCT/ISA/210 (second sheet) (July 2019)

**INTERNATIONAL SEARCH REPORT**

| International application No. |
| --- |
| **PCT/KR2021/011575** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
Information on patent family members

International application No.

**PCT/KR2021/011575**

| Patent document cited in search report | | | Publication date (day/month/year) | Patent family member(s) | | | Publication date (day/month/year) |
|---|---|---|---|---|---|---|---|
| WO | 2019-246379 | A1 | 26 December 2019 | CN | 112584851 | A | 30 March 2021 |
| | | | | EP | 3810171 | A1 | 28 April 2021 |
| | | | | JP | 2021-528104 | A | 21 October 2021 |
| | | | | KR | 10-2021-0033994 | A | 29 March 2021 |
| KR | 10-2020-0032009 | A | 25 March 2020 | CN | 111971295 | A | 20 November 2020 |
| | | | | EP | 3715367 | A1 | 30 September 2020 |
| | | | | JP | 2021-511081 | A | 06 May 2021 |
| | | | | KR | 10-2021-0008110 | A | 20 January 2021 |
| | | | | KR | 10-2201086 | B1 | 11 January 2021 |
| | | | | US | 2020-0369740 | A1 | 26 November 2020 |
| | | | | WO | 2020-060122 | A1 | 26 March 2020 |
| WO | 2019-213517 | A1 | 07 November 2019 | CN | 110437339 | A | 12 November 2019 |
| | | | | CN | 110437339 | B | 13 August 2021 |
| | | | | CN | 112585161 | A | 30 March 2021 |
| | | | | EP | 3788068 | A1 | 10 March 2021 |
| | | | | JP | 2021-522786 | A | 02 September 2021 |
| | | | | KR | 10-2021-0028150 | A | 11 March 2021 |
| WO | 2005-014642 | A2 | 17 February 2005 | EP | 1648931 | A2 | 26 April 2006 |
| | | | | EP | 1648931 | B1 | 09 February 2011 |
| | | | | EP | 1925626 | A1 | 28 May 2008 |
| | | | | EP | 1944318 | A1 | 16 July 2008 |
| | | | | EP | 1944318 | B1 | 02 March 2011 |
| | | | | JP | 2007-523622 | A | 23 August 2007 |
| | | | | JP | 2011-045375 | A | 10 March 2011 |
| | | | | JP | 4842128 | B2 | 21 December 2011 |
| | | | | JP | 4880060 | B2 | 22 February 2012 |
| | | | | US | 2005-0063945 | A1 | 24 March 2005 |
| | | | | US | 2007-0110713 | A1 | 17 May 2007 |
| | | | | US | 2010-0074869 | A1 | 25 March 2010 |
| | | | | US | 2010-0196324 | A1 | 05 August 2010 |
| | | | | US | 7534585 | B2 | 19 May 2009 |
| | | | | US | 7608267 | B2 | 27 October 2009 |
| | | | | US | 7947288 | B2 | 24 May 2011 |
| | | | | WO | 2005-014642 | A3 | 26 May 2005 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8124084 B

- JP 6073417 B

### Non-patent literature cited in the description

- **DUNNE J et al.** *Immunology,* 2001, vol. 167, 3129
- **MR GOODIER et al.** *Immunology,* 2000, vol. 165, 139
- **CONDIOTTI R. et al.** *Experimental Hematology,* 2001, vol. 29, 104
- **BERG, M. et al.** *Cytotherapy,* 2009, vol. 11 (3), 341-55
- **BAEK, H. J. et al.** *Anticancer Res.,* 2013, vol. 33 (5), 2011-9
- **DENMAN, C. J. et al.** *PLoS ONE.,* 2012, vol. 7 (1), e30264
- *J. Immunol.,* 2007, vol. 178 (1), 85-94

- *Cytotherapy,* 2009, vol. 11 (3), 341-355
- **CHILDS, R. W. et al.** *Hematology Am. Soc. Hematol. Educ. Program.,* 2013, vol. 2013 (1), 234-46
- **NI, J. et al.** *OncoImmunology,* 2013, vol. 2 (4), e23811
- **ENGELS ; UHLMANN.** *Angew Chem IntEd Engl,* 1988, vol. 37, 73-127
- **GILLIES et al.** *J. Immunol. Meth.,* 1989, vol. 125, 191-202
- **SAKANO et al.** *Nature,* 1980, vol. 286, 676-683
- **WATSON et al.** *Nucleic Acid Research,* 1984, vol. 12, 5145-5164